# EUROPÄISCHE PATENTSCHRIFT

(11) **EP 3 232 059 B1**
(45) Veröffentlichungstag und Bekanntmachung des Hinweises auf die Patenterteilung: **03.07.2024**
(21) Anmeldenummer: 17157555.8
(22) Anmeldetag: 23.02.2017
(51) Int. Cl.: F04B 43/00, F04B 43/12, F04B 17/03, A61M 5/142, F04B 53/16

(54) **SCHLAUCHPUMPE**
HOSE PUMP
POMPE TUBULAIRE

(30) Priorität: 11.04.2016 DE 202016101907 U
(43) Veröffentlichungstag der Anmeldung: 18.10.2017
(73) Patentinhaber: Ulrich GmbH & Co. KG, 89081 Ulm (DE)
(72) Erfinder: Bückle, Norbert, 89182 Bernstadt (DE)
(74) Vertreter: Charrier Rapp & Liebau

(56) Entgegenhaltungen:
- EP-A1- 0 041 267
- EP-A2- 2 924 288
- WO-A2-2006/110510
- DE-A1- 2 162 998
- DE-A1-102014 004 476
- JP-A- 2012 233 427

## Beschreibung

Die Erfindung betrifft eine Schlauchpumpe nach dem Oberbegriff des Anspruchs 1.

Derartige Schlauchpumpen sind beispielsweise aus der DE 10 2014 104 320 B1 und der DE 102010 000 594 A1 bekannt. Diese bekannten Schlauchpumpen verfügen über ein Schlauchbett, in das ein schlaufenförmig gebogener Schlauchabschnitt eines Schlauchs eingelegt werden kann. Die bekannten Schlauchpumpen umfassen weiterhin ein Gegenlager und eine relativ zum Gegenlager drehbare Trägerscheibe, auf deren Oberseite einer Mehrzahl von Quetschrollen und eine Mehrzahl von Führungsrollen angeordnet sind. Dabei sind sowohl die Quetschrollen als auch die Führungsrollen im radial äußeren Bereich der Trägerscheibe und jeweils in Umfangsrichtung der Trägerscheibe jeweils äquidistant zueinander angeordnet, wobei zwischen zwei in Umfangsrichtung der Trägerscheibe aufeinanderfolgende Quetschrollen jeweils eine Führungsrolle angeordnet ist. In einem Ausführungsbeispiel der bekannten Schlauchpumpen sind bspw. je drei Quetschrollen und Führungsrollen vorgesehen, die in Umfangsrichtung der Trägerscheibe jeweils einen Winkelabstand von 60° zur benachbarten Quetschrolle bzw. Führungsrolle aufweisen. Die Quetschrollen weisen einen glatten Außenumfang auf und drücken bei sich in einer Förderrichtung drehender Trägerscheibe einen in das Schlauchbett eingelegten Schlauch unter Quetschung des Schlauchs gegen das Gegenlager, um ein sich in dem Schlauch befindliches Fluid in Förderrichtung zu transportieren. Die zylindrischen Führungsrollen weisen an ihrem Außenumfang eine in Umfangsrichtung umlaufende Führungsnut zur Aufnahme der radial inneren Schlauchhälfte des Schlauchabschnitts auf und sorgen sowohl beim Einfädeln des Schlauchabschnitts in das Schlauchbett als auch während des Pumpbetriebs für eine exakte Positionierung und Führung des Schlauchs in dem Schlauchbett.

Eine gattungsgemäße Schlauchpumpe mit einer drehbaren Rollenanordnung, die mindestens zwei Quetschrollen und wenigstens eine Führungsrolle umfasst, welche jeweils peripher und drehbar auf der Rollenanordnung angeordnet sind, ist in der WO 2006/110510 A1 offenbart. Eine weitere Schlauchpumpe ist in JP 2012-233427 A gezeigt.

Zum automatisierten Einfädeln des Schlauchabschnitts in das Schlauchbett kann eine motorisch betrieben Einfädeleinrichtung verwendet werden wie sie bspw. in der EP 2 542 781 A1 beschrieben ist. Alternativ dazu kann der Schlauchabschnitt zum Einfädeln in das Schlauchbett auch mit einem Niederhalter gegen eine Auflagefläche am Eingang des Schlauchbetts gedrückt und bei sich drehender Trägerscheibe von einer der Führungsrollen ergriffen und dabei in das Schlauchbett gezogen werden, wobei der radial innere Bereich des Schlauchabschnitts in der Führungsnut der Führungsrolle aufgenommen und in axialer Richtung nach unten auf eine Auflagefläche in dem Schlauchbett gedrückt wird. Dabei kann es zu Problemen kommen, wenn der Schlauchabschnitt zu kurz ist. Es besteht dann die Gefahr, dass der zu kurze Schlauchabschnitts beim Einfädeln gedehnt und unter Zugspannung gesetzt wird und dadurch aus der Führungsnut der Führungsrolle herausrutscht.

Wenn der im Schlauchbett eingelegte Schlauchabschnitt zu lang ist, kann es beim Betrieb der bekannten Schlauchpumpen zu Problemen kommen, weil der Schlauchabschnitt am Ausgang des Schlauchbetts eine über der Auflagefläche des Schlauchbetts vorstehende Schlaufe ausbildet und deshalb nicht sauber im Schlauchbett geführt wird. Insbesondere bei sehr hohen Pumpdrücken, die beim bestimmungsgemäßen Betrieb der Schlauchpumpe bis zu 20 bar erreichen können, besteht dadurch die Gefahr, dass das stromabwärtige Ende des in das Schlauchbett eingelegten Schlauchabschnitts aus der Führungsnut der Führungsrollen herausrutscht und sich dadurch von der Auflagefläche des Schlauchbetts abhebt. Dies kann dazu führen, dass sich der Schlauchabschnitt während des Betriebs der Schlauchpumpe selbsttätig und ungewollt ausfädelt.

Hiervon ausgehend liegt der Erfindung die Aufgabe zugrunde, eine gattungsgemäße Schlauchpumpe so weiter zu bilden, dass eine zuverlässige Einfädelung eines Schlauchabschnitts eines Pumpenschlauchs auch dann sicher gestellt werden kann, wenn der Schlauchabschnitt im Vergleich zum Innenumfang des Gegenlagers etwas zu kurz sein sollte. Weiterhin soll verhindert werden, dass sich beim Betrieb der Schlauchpumpe, insbesondere unter hohen Pumpdrücken, der eingelegte Schlauchabschnitt selbsttätig ausfädelt und es soll die Lebensdauer des Pumpenschlauchs verlängert werden.

Diese Aufgaben werden mit einer Schlauchpumpe mit den Merkmalen des Anspruchs 1 gelöst. Bevorzugte Ausführungsformen dieser Schlauchpumpe sind den Unteransprüchen zu entnehmen.

Die erfindungsgemäße Schlauchpumpe verfügt über ein Schlauchbett zum Einlegen eines Schlauchabschnitts eines Pumpschlauchs, ein Gegenlager, eine relativ zum Gegenlager drehbare Trägerscheibe, eine Mehrzahl von in Umfangsrichtung auf der Trägerscheibe äquidistant zueinander angeordnete Quetschrollen und eine Mehrzahl von in Umfangsrichtung auf der Trägerscheibe äquidistant zueinander angeordnete Führungsrollen, wobei zwischen zwei in Umfangsrichtung der Trägerscheibe aufeinanderfolgende Quetschrollen jeweils eine Führungsrolle angeordnet ist und die Quetschrollen bei sich in einer Förderrichtung drehender Trägerscheibe einen in das Schlauchbett eingelegten Schlauch (Schlauchabschnitt) unter Quetschung des Schlauchs gegen das Gegenlager drücken, um ein in dem Schlauch befindliches Fluid in Förderrichtung zu transportieren.

Im Zentrum der Trägerscheibe ist dabei koaxial zur Drehachse ein über der Oberfläche der Trägerscheibe vorstehender Zylinder angeordnet, dessen Außendurchmesser annähernd bis zum Außenumfang der radial weiter außen liegenden Quetschrollen und Führungsrollen reicht, wobei der radiale Abstand zwischen der Mantelfläche des Zylinders und dem Außenumfang der Quetschrollen bzw. der Führungsrollen kleiner ist als der Durchmesser des in das Schlauchbett (2) eingelegten Schlauchs.

Anders als bei den oben zitierten bekannten Schlauchpumpen sind die Quetschrollen und die Führungsrollen in der erfindungsgemäßen Schlauchpumpe nicht alle in einem äquidistanten Abstand zueinander und symmetrisch über den Umfang der Trägerscheibe verteilt angeordnet. In der erfindungsgemäßen Schlauchpumpe sind die Führungsrollen jeweils in Bezug auf die ihnen in Förderrichtung (Drehrichtung der Trägerscheibe beim Pumpbetrieb der Schlauchpumpe) nachfolgenden Quetschrollen zurück versetzt, d.h. der Winkelabstand (δ) zwischen einer Führungsrolle und der in Förderrichtung dieser Führungsrolle nachfolgenden Quetschrolle ist kleiner ist als der Winkelabstand (Δ) zwischen dieser Führungsrolle und der in Förderrichtung dieser Führungsrolle vorausgehenden Quetschrolle.

Durch diese Anordnung der Quetschrollen und der Führungsrollen auf der Trägerscheibe wird beim Einfädeln des Schlauchs in das Schlauchbett verhindert, dass der stromaufwärtige Abschnitt des Schlauchs aus der Führungsnut einer Führungsrolle herausrutschen kann, weil der Führungsrolle beim Drehen der Trägerscheibe unmittelbar, d.h. unter nur geringen Winkelabstand δ, eine Quetschrolle nachfolgt, welche den stromaufwärtigen Abschnitt des Schlauchs gegen das Gegenlager drückt und dadurch die Position des schon in das Schlauchbett eingeführten Abschnitt des Schlauchs im Schlauchbett fixiert.

Beim Pumpbetrieb der Schlauchpumpe wird durch die erfindungsgemäße Anordnung der Quetschrollen und der Führungsrollen auf der Trägerscheibe ein ungewolltes Ausfädeln des Schlauchs verhindert, weil jeder Quetschrolle beim Drehen der Trägerscheibe unmittelbar, d.h. unter nur geringem Winkelabstand δ, eine Führungsrolle vorauseilt, welche den stromabwärtigen Abschnitt des Schlauchs auch bei hohen Pumpdrücken sicher im Schlauchbett hält und verhindert, dass sich das stromabwärtige Ende des Schlauchs am Ausgang des Schlauchbetts zu einer Schlaufe aufwölben kann, während der etwas weiter in Förderrichtung gesehen zurück liegende Abschnitt des Schlauchs von der Quetschrolle gegen das Gegenlager gepresst wird.

Bevorzugt liegt der Betrag der relativen Winkeldifferenz (Δ - δ / Δ + δ) zwischen dem Winkelabstand Δ zwischen einer Führungsrolle und der in Förderrichtung dieser Führungsrolle vorausgehenden Quetschrolle und dem Winkelabstand δ zwischen dieser Führungsrolle und der in Förderrichtung dieser Führungsrolle nachfolgenden Quetschrolle im Bereich von 0,2 bis 0,5.

Zweckmäßig sind die Führungsrollen und die Quetschrollen drehsymmetrisch (in Bezug auf die Drehachse der Trägerschreibe als Symmetriezentrum) auf der Trägerscheibe verteilt angeordnet, wobei der Symmetriewinkel 360°/n ist, wenn n die Anzahl der Führungsrollen bzw. der Quetschrollen ist.

In einer bevorzugten Ausführungsform weist die erfindungsgemäße Schlauchpumpe drei oder mehr Quetschrollen und eine gleiche Anzahl von Führungsrollen auf, welche so am radial äußeren Rand der Trägerscheibe angeordnet sind, dass der Winkelabstand (δ) zwischen jeder Führungsrolle und der in Förderrichtung einer Führungsrolle nachfolgenden Quetschrolle kleiner als 60° ist und insbesondere - bei drei Führungsrollen und drei Quetschrollen - bevorzugt 45° beträgt. In entsprechender Weise ist der Winkelabstand (Δ) zwischen einer Führungsrolle und der in Förderrichtung dieser Führungsrolle vorausgehenden Quetschrolle größer als 60° ist und beträgt insbesondere mindestens 75°. Bei dieser Anordnung mit drei Quetschrollen und drei Führungsrollen liegt der Betrag der relativen Winkeldifferenz bevorzugt bei Δ - δ / Δ + δ = 0,25. Bei einer alternativen Anordnung mit vier Quetschrollen und vier Führungsrollen liegt der Betrag der relativen Winkeldifferenz bevorzugt bei Δ - δ / Δ + δ = 0,33.

Gemäß der Erfindung ist im Zentrum der Trägerscheibe koaxial zu deren Drehachse ein über der Oberfläche der Trägerscheibe vorstehender Zylinder angeordnet, dessen Außendurchmesser zumindest annähernd bis zum Außenumfang der radial weiter außen liegenden Quetschrollen und Führungsrollen reicht. Der in Bezug auf die Führungsrollen und die Quetschrollen auf der Trägerscheibe radial innen liegende Zylinder verhindert beim Einfädeln des Schlauchs, dass ein Abschnitt des Schlauchs an einem radial inneren Abschnitt (in Bezug auf die Trägerscheibe gesehen) des Außenumfangs der Führungsrollen zu liegen kommen kann und deshalb von dieser Führungsrolle nicht richtig erfasst und in das Schlauchbett zwischen dem Außenumfang der Führungsrolle und dem Gegenlager eingeführt werden kann. Hierfür ist es zweckmäßig, wenn der radiale Abstand zwischen der Mantelfläche des Zylinders und dem Außenumfang der Führungsrollen kleiner ist als der Durchmesser des in das Schlauchbett einzuführenden Schlauchs.

Um die Trägerscheibe beim Betrieb der Pumpe in Drehung zu versetzen, ist die Trägerscheibe mit einer Welle verbunden, welche mit einem Antrieb gekoppelt ist und von diesem in Rotation versetzt werden kann. Die Führungsrollen und die Quetschrollen sind bevorzugt drehbar auf der Trägerscheibe gelagert, um ein reibungsfreies Abrollen an der Oberfläche des Schlauchs zu ermöglichen. Sie können aber auch jeweils drehfest mit der Trägerscheibe verbunden sein. Die Drehachse der Trägerscheibe (Achse der Welle) und die Achsen der Quetschrollen und der Führungsrollen verlaufen dabei parallel zueinander. Wenn die Führungsrollen und die Quetschrollen drehbar auf der Trägerscheibe gelagert sind, können sie (ggf. über ein Getriebe) von einem Antrieb in Rotation versetzt, wobei es sich bei dem Antrieb 7 bevorzugt um den Antrieb handelt, der auch die Trägerscheibe drehend antreibt. Die Führungsrollen und die Quetschrollen können jedoch auch ohne Kopplung an einen Antrieb drehbar auf der Trägerscheibe gelagert sein.

Die Quetschrollen sind zweckmäßig zumindest im Wesentlichen zylindrisch und mit einer glatten Mantelfläche ausgebildet, wobei der Außenumfang der Quetschrollen, der den Schlauch gegen das Gegenlager presst, durch die glatte Mantelfläche gebildet ist. Die Führungsrollen weisen an ihrem Außenumfang zweckmäßig eine umlaufende Führungsnut auf, welche der Form des Schlauchs angepasst ist und im Querschnitt bspw. für einen Schlauch mit kreisförmigem Querschnitt halbkreisförmig ausgebildet ist. Aufgrund der Ausformung der Führungsnut am Außenumfang der Führungsrollen schmiegen sich diese bei laufender Schlauchpumpe an die Oberfläche des Schlauchs an, ohne diesen zu quetschen. Dadurch wird bei laufender Schlauchpumpe eine sichere und gleichbleibende Führung des Schlauchs im Schlauchbett gewährleistet.

Diese und weitere Vorteile und Merkmale der erfindungsgemäßen Schlauchpumpe ergeben sich aus dem nachfolgend unter Bezugnahme auf die begleitenden Zeichnungen näher beschriebenen Ausführungsbeispiel. Die Zeichnungen zeigen:
**Figur 1****:** Perspektivische Darstellung einer erfindungsgemäßen Schlauchpumpe mit darin eingelegtem Schlauch;
**Figur 2****:** Querschnitt der Schlauchpumpe von Figur 1 (ohne Schlauch);

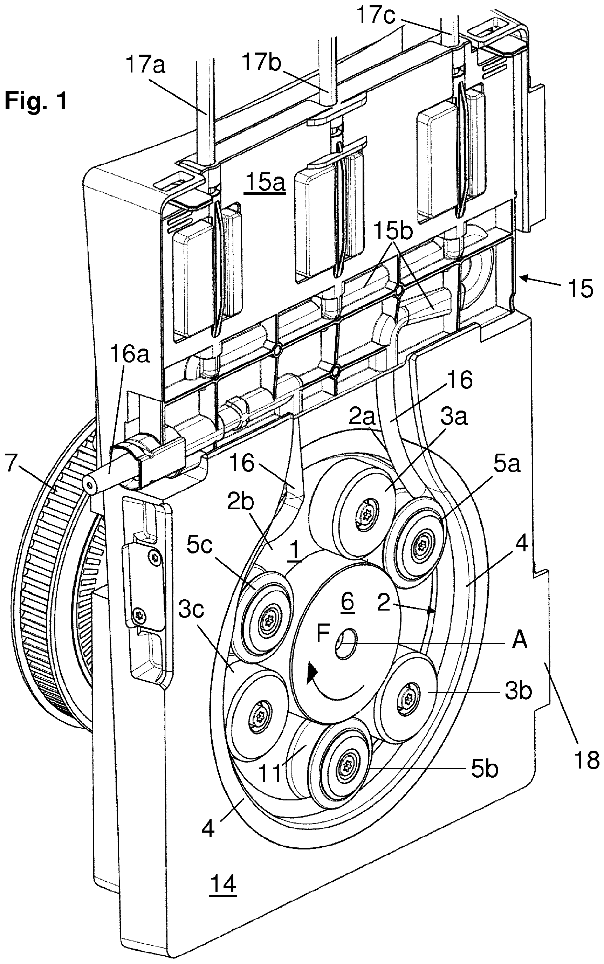

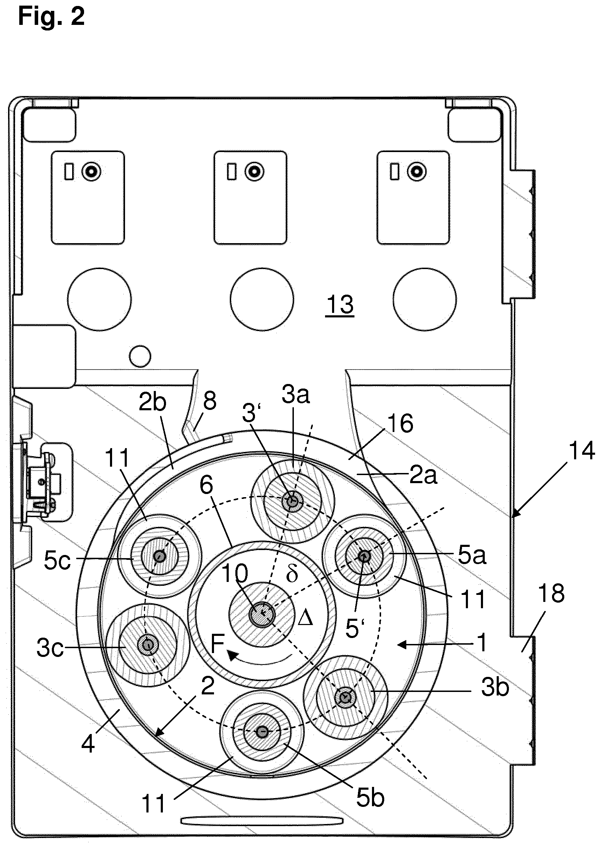

In Figur 1 und Figur 2 ist ein Ausführungsbeispiel einer erfindungsgemäßen Schlauchpumpe in einer perspektivischen Darstellung (mit eingelegtem Schlauch 16) bzw. einer Schnittansicht (ohne Schlauch) gezeigt. Die Schlauchpumpe dient zur Förderung eines in einem Schlauch 16 geführten Fluids, bspw. einer Injektionsflüssigkeit für eine medizinische, insbesondere intravenöse Injektion. Die Schlauchpumpe ist in einem Pumpengehäuse 14 angeordnet, an dem ein verschwenkbarer und aus Gründen der besseren Übersichtlichkeit hier nicht dargestellter Gehäusedeckel mittels einer Befestigungseinrichtung 18 angelenkt ist.

Die Schlauchpumpe umfasst eine Trägerscheibe 1, welche über eine zentral in der Trägerscheibe 1 angeordnete Antriebswelle 10 mit einem Antrieb 7 gekoppelt ist. Bei dem Antrieb 7 handelt es sich bspw. um einen Elektromotor. Die Trägerscheibe 1 wird bei laufendem Antrieb 7 über die drehfest mit der Trägerscheibe 1 verbundene Antriebswelle 10 um eine Drehachse A in Förderrichtung (F) in Drehung versetzt. Bei dem zeichnerisch dargestellten Ausführungsbeispiel verläuft die Förderrichtung F (Drehrichtung der Trägerscheibe im Pumpbetrieb) im Uhrzeigersinn.

Die Schlauchpumpe umfasst weiterhin ein Schlauchbett 2 mit einem Schlaucheingang 2a und einem Schlauchausgang 2b, sowie ein Gegenlager 4. Das Gegenlager 4 ist vom Innenumfang eines Kreissegments gebildet, welches im Bereich des Schlaucheingangs 2a und des Schlauchausgangs 2b des Schlauchbetts 2 zur Einführung eines Schlauchs 16 offen ist. Das Schlauchbett 2 dient zur Aufnahme eines Schlauchabschnitts eines Pumpenschlauchs (der Schlauchabschnitt wird im Folgenden allgemein als Schlauch bezeichnet), in dem ein Fluid (beispielsweise eine Injektionsflüssigkeit zur intravenösen Injektion in die Blutbahn eines Patienten) geführt wird. Ein in das Schlauchbett 2 eingelegter Schlauch liegt dabei auf einer von der Oberfläche der Trägerscheibe 1 gebildeten Führungsfläche 2c auf. Im Bereich des Schlauchausgangs 2b des Schlauchbetts 2 läuft das Gegenlager 4 tangential nach außen aus, wie aus den Figuren ersichtlich.

Auf der Oberfläche der Trägerscheibe 1 sind im radial äußeren Abschnitt (nahe ihres Außenumfangs) mehrere Quetschrollen 3 angeordnet. Die Achsen 3' der Quetschrollen liegen dabei auf einer konzentrisch zur Drehachse (A) der Trägerscheibe 1 verlaufenden Kreisbahn (gestrichelte Linie in Figur 2). Bei dem hier zeichnerisch dargestellten Ausführungsbeispiel der erfindungsgemäßen Schlauchpumpe sind drei solcher Quetschrollen 3a, 3b, 3c vorgesehen und gleichmäßig über den Umfang der Trägerscheibe 1 verteilt angeordnet. Wenn im Folgenden auf die jeweils gleich ausgebildeten Quetschrollen 3a, 3b, 3c Bezug genommen wird erfolgt dies mit dem Bezugszeichen 3. Die Quetschrollen 3 sind zumindest im Wesentlichen zylindrisch mit einer glatten Mantelfläche ausgebildet.

Zwischen benachbarten Quetschrollen 3 ist jeweils eine Führungsrolle 5 auf der Trägerscheibe 1 angeordnet. Die Achsen 5' der Führungsrollen 5 liegen dabei ebenfalls auf der konzentrisch zur Drehachse (A) der Trägerscheibe 1 verlaufenden Kreisbahn (gestrichelte Linie in Figur 2). Bei dem hier zeichnerisch dargestellten Ausführungsbeispiel der erfindungsgemäßen Schlauchpumpe sind drei solcher Führungsrollen 5a, 5b, 5c vorgesehen und gleichmäßig über den Umfang der Trägerscheibe 1 (bzw. auf der gestrichelten Kreisbahn) verteilt angeordnet. Wenn im Folgenden auf die jeweils gleich ausgebildeten Führungsrollen 5a, 5b, 5c Bezug genommen wird, erfolgt dies mit dem Bezugszeichen 5.

Die Führungsrollen 5 weisen eine zylindrische Grundform und an ihrem Außenumfang (am Zylindermantel) eine in Umfangsrichtung umlaufende Führungsnut 11 auf. Sowohl die Quetschrollen 3 als auch die Führungsrollen 5 sind zweckmäßig drehbar auf der Trägerscheibe 1 gelagert, wobei die Drehachsen 3' der Quetschrollen 3 und die Drehachsen 5' der Führungsrollen 5 jeweils parallel zur Antriebswelle 10 verlaufen. Die Quetschrollen 3 und die Führungsrollen 5 können dabei entweder frei drehbar auf der Trägerscheibe 1 gelagert sein oder auch über eine Kupplung mit dem Antrieb 7 gekoppelt sein. Wenn die Quetschrollen 3 und/oder die Führungsrollen 5 über eine Kupplung mit dem Antrieb 7 gekoppelt sind, werden sie bei laufendem Antrieb 7 von diesem gleichsinnig zur Trägerscheibe 1 in Drehung versetzt (im Uhrzeigersinn).

Die drei Quetschrollen 3a, 3b, 3c und die drei Führungsrollen 5a, 5b, 5c sind so am radial äußeren Rand der Trägerscheibe 1 angeordnet, dass der Winkelabstand δ zwischen jeder Führungsrolle und der in Förderrichtung einer Führungsrolle nachfolgenden Quetschrolle kleiner als 60° ist und - wie in dem gezeigten Ausführungsbeispiel der Figuren 1 und 2 - insbesondere 45° beträgt. In entsprechender Weise ist der Winkelabstand Δ zwischen einer Führungsrolle und der in Förderrichtung dieser Führungsrolle vorausgehenden Quetschrolle größer als 60° und beträgt in dem gezeigten Ausführungsbeispiel 75°. In dem in den Figuren 1 und 2 gezeigten Ausführungsbeispiel beträgt also der Winkelabstand δ zwischen der Führungsrolle 5a und der in Förderrichtung F dieser Führungsrolle 5a nachfolgenden Quetschrolle (3a) δ = 45°. In entsprechender Weise ist der Winkelabstand Δ zwischen der Führungsrolle 5a und der in Förderrichtung dieser Führungsrolle 5a vorausgehenden Quetschrolle (3b) bei Δ = 75°.

Ein in das Schlauchbett 2 eingelegter Schlauch wird von den Führungsrollen 5 geführt, indem der Schlauch in die umfangsseitigen Führungsnuten 11 der Führungsrollen eingreift. Dadurch wird der Schlauch auf der von der Oberfläche der Trägerscheibe 1 gebildeten Führungsfläche 2c gehalten und es wird verhindert, dass der Schlauch bei laufender Pumpe aus dem Schlauchbett 2 herausrutschen kann.

Im Zentrum der Trägerscheibe 1 ist koaxial zu deren Drehachse A ein über der Oberfläche der Trägerscheibe 1 vorstehender Zylinder 6 angeordnet, der die Antriebswelle 10 umgibt und dessen (Außen-)Durchmesser D zumindest annähernd bis zum Außenumfang der radial weiter außen liegenden Quetschrollen und Führungsrollen reicht, d.h. zwischen dem Außenumfang des Zylinders 6 und dem Außenumfang der Quetschrollen und Führungsrollen besteht ein (möglichst) kleiner Abstand (Figur 1). Der Zylinder 6 kann, wie aus Figur 2 ersichtlich, als Hohlzylinder oder auch als Vollzylinder ausgebildet sein Der Zylinder 6 ist zweckmäßig drehfest mit der Trägerscheibe 1 verbunden. Der Zylinder 6 verhindert beim Einfädeln des Schlauchs, dass dieser auf der radial nach innen weisenden Seite der Führungsrollen 5 zu liegen kommt und daher nicht ordentlich in das Schlauchbett 2 zwischen dem Außenumfang der Quetschrollen 3 und dem Gegenlager 4 eingefädelt werden kann. Hierfür sollte der radiale Abstand zwischen der Mantelfläche des Zylinders und dem Außenumfang der Führungsrollen kleiner sein als der Durchmesser des in das Schlauchbett einzuführenden Schlauchs. Die Höhe des Zylinders 6 (in axialer Richtung) ist dabei zweckmäßig an die Höhe der Quetschrollen und der Führungsrollen angepasst und weist mindestens dieselbe Höhe auf wie die Quetschrollen und die Führungsrollen. Zweckmäßig überragt der Zylinder 6 die Quetschrollen und die Führungsrollen in axialer Richtung etwas, um ein ungehindertes Eingleiten des Schlauchs in das Führungsbett 2 zu ermöglichen.

Das Gehäuse 14 der Pumpe enthält eine als Vertiefung im Gehäuse ausgebildete Kassettenaufnahme 13 (Figur 2) zum Einsetzen einer austauschbaren Kassette 15 (Figur 1). In der in Figur 1 gezeigten Kassette 15 ist ein Führungskanal 15b sowie ein damit verbundener Pumpenschlauch 16 integriert, in denen das zu fördernde Fluid geführt wird. Ein schlaufen- bzw. bogenförmiger Abschnitt des Pumpenschlauchs ragt dabei aus einem Gehäuse 15a der Kassette heraus. An der Oberseite der Kassette 15 sind mehrere Verbindungsschläuche 17a, 17b, 17c angeordnet, welche mit Vorratsflaschen für zu injizierende Flüssigkeiten (bspw. Kontrastmittel) verbunden werden können. Die Verbindungsschläuche 17a, 17b, 17c sind über den Führungskanal 15b mit dem Pumpenschlauch 16 verbunden. Seitlich ist an der Kassette 15 ein Verbinder 16a angeordnet, an den ein Patientenschlauch angeschlossen werden kann, um diesen mit dem Pumpenschlauch 16 zu verbinden.

Am Schlauchausgang 2b ist eine Ausfädeleinrichtung mit einer über der Oberfläche der Trägerscheibe 1 vorstehenden Erhebung 8 angeordnet, wie aus der DE 10 2014 104 320 B3 bekannt, welche hierzu in Bezug genommen wird.

Für den Betrieb der Schlauchpumpe wird der aus der Kassette herausragende Abschnitt des Pumpenschlauchs in das Schlauchbett 2 eingeführt, wobei der Schlauch durch die Führungsrollen 5 geführt wird und dabei mit geringem Abstand und im Wesentlichen parallel zur Oberfläche der Trägerscheibe 1 sowie zwischen dem Außenumfang der Quetschrollen 3 und dem Gegenlager 4 bzw. zwischen der Führungsnut 11 der Führungsrollen 5 und dem Gegenlager 4 verläuft. Der (radiale) Abstand zwischen dem Außenumfang der Quetschrollen 3 ist dabei kleiner gewählt als der Durchmesser des Schlauchs, so dass der Schlauch zwischen dem Außenumfang der Quetschrollen 3 und dem Gegenlager 4 unter Quetschung des flexiblen Schlauchs eingeklemmt wird.

Beim Pumpbetrieb der Schlauchpumpe wird die Trägerscheibe 1 (und ggf. über ein Getriebe auch die darauf angeordneten Quetschrollen 3 und die Führungsrollen 5) von dem Antrieb 7 in Förderrichtung F in Drehung versetzt. In dem in den Figuren gezeigten Ausführungsbeispiel wird die Trägerscheibe 1 im Pumpbetrieb im Uhrzeigersinn gedreht. Dabei wird der im Schlauchbett 2 liegende Abschnitt des Schlauchs von den Quetschrollen 3 gegen das Gegenlager 4 gepresst, wodurch der Schlauch intermittierend gequetscht und das sich in dem Schlauch befindliche Fluid in Richtung vom Schlaucheingang 2a zum Schlauchausgang 2b gefördert wird. Die Führungsrollen 5 stellen dabei eine sichere und gleichbleibende Positionierung des Abschnitts des Schlauchs in dem Schlauchbett 2 sicher, indem der Schlauch in die Führungsnut 11 der Führungsrollen 5 eingreift und dadurch geführt wird.

Zum Einfädeln des aus der Kassette herausragenden Abschnitts des Schlauchs ist im Bereich des Schlaucheingangs 2a zweckmäßig eine Einfädeleinrichtung vorgesehen. Diese Einfädeleinrichtung kann durch eine motorisch angetriebene Schneckenspindel gebildet sein, wie sie aus der DE 10 2010 000 594 B4 bekannt ist, welche hierfür in Bezug genommen wird. Eine kostengünstigere Einfädeleinrichtung, welche auf die Verwendung einer motorisch angetriebenen Schneckenspindel verzichtet, ist in der DE 102014 104320 A1 beschrieben, welche hierfür in Bezug genommen wird.

Beim Einfädeln des Schlauchs in das Schlauchbett wird durch die erfindungsgemäße Anordnung der Quetschrollen 3 und der Führungsrollen 5 auf der Trägerscheibe 1 verhindert, dass der stromaufwärtige Abschnitt des Schlauchs aus der Führungsnut 11 der beim Einfädeln im Bereich des Schlaucheingangs 2a liegenden Führungsrolle (in Figur 1 und 2 ist dies die Führungsrolle 5a) herausrutschen kann. Die dieser Führungsrolle (5a) beim Drehen der Trägerscheibe unmittelbar unter dem geringen Winkelabstand δ nachfolgende Quetschrolle (3a) drückt nämlich den stromaufwärtigen Abschnitt des Schlauchs, der bereits von der vorauseilenden Führungsrolle (5a) eingefädelt worden ist, gegen das Gegenlager 4 und fixiert dadurch die Position des schon in das Schlauchbett eingeführten Abschnitts des Schlauchs im Schlauchbett 2. Dadurch wird verhindert, dass der ggf. etwas zu kurze Schlauch beim Einfädeln zu stark gedehnt und dadurch aus der Führungsnut 11 der Führungsrolle 5a herausrutschen kann.

Nach dem Einfädeln des aus der Kassette herausragenden Abschnitts des Schlauchs in das Schlauchbett 2 in der in der DE 102014 104320 A1 beschriebenen Weise (welche hierfür in Bezug genommen wird) kann die Pumpe zur Förderung des sich im Schlauch befindlichen Fluids in ihrer Förderrichtung F betrieben werden. Hierfür wird die Trägerscheibe 1 bei dem hier zeichnerisch dargestellten Ausführungsbeispiel von dem Antrieb 7 im Uhrzeigersinn in Drehung versetzt, wodurch die Quetschrollen 3 den Schlauch unter Quetschung gegen das Gegenlager 4 drücken und dadurch das im Schlauch befindliche Fluid in Förderrichtung transportieren.

Durch die Führung des Schlauchs in der erfindungsgemäßen Schlauchpumpe wird im Bereich des Schlauchausgang 2b des Schlauchbetts 2 gewährleistet, dass die im Pumpbetrieb dort vorbeilaufende Quetschrolle (in den Zeichnungen ist dies die Quetschrolle 3c) den dort eingelegten Schlauchabschnitt erst dann überfährt, wenn dieser aufgrund des dort tangential nach außen auslaufenden Gegenlagers 4 bereits vollständig entlastet ist. Dadurch wird die Lebensdauer des Schlauchs verlängert. Bei den bekannten Schlauchpumpen besteht die Gefahr, dass eine Quetschrolle den Schlauch im Bereich des Schlauchausgang des Schlauchbetts quer zur Förderrichtung F überfährt und noch gegen das Gegenlager 4 presst, wodurch es zu einer erhöhten Walkarbeit und damit zu erhöhter mechanischer Belastung am Material des Schlauchs kommt. Durch die höhere mechanische Belastung verliert der Schlauch schneller seine Steifigkeit und muss früher ausgetauscht werden, da er der Druckbelastung nicht mehr stand halten kann.

Die Erfindung ist nicht auf die hier zeichnerisch dargestellte Ausführungsform beschränkt. So kann bspw. die Anzahl der Quetschrollen 3 und der Führungsrollen 5 anders gewählt werden. Zweckmäßig ist es jedoch, gleich viele Führungsrollen und Quetschrollen vorzusehen, also bspw. vier Quetschrollen 3 und vier Führungsrollen 5, welche in abwechselnder Reihenfolge so auf der Trägerscheibe 1 angeordnet sind, dass ihre Achsen auf einer konzentrisch um die Drehachse A der Trägerscheibe 1 verlaufenden Kreisbahn liegen. Dabei sind die Winkelabstände zwischen den Quetschrollen untereinander und zwischen den Führungsrollen untereinander äquidistant. Bei je vier Führungs- und Quetschrollen beträgt dieser Abstand zwischen den Führungs- bzw. Quetschrollen jeweils 90°.

## Patentansprüche

1. Schlauchpumpe zur Förderung eines in einem Schlauch (16) geführten Fluids, mit einem ein Gegenlager (4) aufweisendes Schlauchbett (2), einer relativ zum Gegenlager (2) drehbaren Trägerscheibe (1), einer Mehrzahl von in Umfangsrichtung auf der Trägerscheibe (1) äquidistant zueinander angeordnete Quetschrollen (3) und einer Mehrzahl von in Umfangsrichtung auf der Trägerscheibe (1) äquidistant zueinander angeordnete Führungsrollen (5), wobei zwischen zwei in Umfangsrichtung der Trägerscheibe (1) aufeinanderfolgende Quetschrollen (3) jeweils eine Führungsrolle (5) angeordnet ist und die Quetschrollen (3) bei sich in einer Förderrichtung (F) drehender Trägerscheibe (1) einen in das Schlauchbett (2) eingelegten Schlauch unter Quetschung des Schlauchs gegen das Gegenlager (4) drücken, um das Fluid in dem Schlauch (16) in Förderrichtung zu transportieren, wobei der Winkelabstand (Δ) zwischen einer Führungsrolle (5a) und der in Förderrichtung (F) dieser Führungsrolle (5a) vorausgehenden Quetschrolle (3b) größer ist als der Winkelabstand (δ) zwischen dieser Führungsrolle (5a) und der in Förderrichtung (F) dieser Führungsrolle (5a) nachfolgenden Quetschrolle (3a), **dadurch gekennzeichnet, dass** im Zentrum der Trägerscheibe (1) koaxial zur Drehachse (A) ein über der Oberfläche der Trägerscheibe (1) vorstehender Zylinder (6) mit einer Mantelfläche angeordnet ist, wobei der Außendurchmesser (D) des Zylinders (6) zumindest annähernd bis zum Außenumfang der radial weiter außen liegenden Quetschrollen (3) und Führungsrollen (5) reicht, und der radiale Abstand zwischen der Mantelfläche des Zylinders (6) und dem Außenumfang der Führungsrollen (5) kleiner ist als der Durchmesser des in das Schlauchbett (2) eingelegten Schlauchs (16).

2. Schlauchpumpe nach Anspruch 1, **dadurch gekennzeichnet, dass** der Winkelabstand (δ) zwischen einer Führungsrolle (5a) und der in Förderrichtung dieser Führungsrolle (5a) nachfolgenden Quetschrolle (3a) kleiner als 60° ist und insbesondere maximal 45° beträgt.

3. Schlauchpumpe nach Anspruch 1, **dadurch gekennzeichnet, dass** der Winkelabstand (Δ) zwischen einer Führungsrolle (5a) und der in Förderrichtung dieser Führungsrolle (5a) vorausgehenden Quetschrolle (3b) größer als 60° ist und insbesondere mindestens 75° beträgt.

4. Schlauchpumpe nach einem der voranstehenden Ansprüche, **dadurch gekennzeichnet, dass** mindestens drei Quetschrollen (3) und mindestens drei Führungsrollen (5) auf der Trägerscheibe (1) angeordnet sind.

5. Schlauchpumpe nach einem der voranstehenden Ansprüche, **dadurch gekennzeichnet, dass** die Quetschrollen (3) zumindest im Wesentlichen zylindrisch und mit einer glatten Mantelfläche ausgebildet sind, wobei der Außenumfang der Quetschrollen (3) durch die glatte Mantelfläche gebildet ist.

6. Schlauchpumpe nach einem der voranstehenden Ansprüche, **dadurch gekennzeichnet, dass** die Führungsrollen (5) zumindest im Wesentlichen zylindrisch sind und an ihrem Außenumfang eine in Umfangsrichtung umlaufende Führungsnut (11) aufweisen.

7. Schlauchpumpe nach Anspruch 6, **dadurch gekennzeichnet, dass** die Führungsnut (11) jeder Führungsrolle (5) einen halbkreisförmigen Querschnitt aufweist.

8. Schlauchpumpe nach einem der voranstehenden Ansprüche, **dadurch gekennzeichnet, dass** ein in das Schlauchbett (2) eingelegter Schlauch auf einer von der Oberfläche der Trägerscheibe (1) gebildeten Führungsfläche (2c) aufliegt und von den Führungsrollen (5) in dieser Position geführt und gehalten wird.

9. Schlauchpumpe nach einem der voranstehenden Ansprüche, **dadurch gekennzeichnet, dass** die Quetschrollen (3) und/oder die Führungsrollen (5) drehbar auf der Trägerscheibe (1) gelagert sind, wobei die Drehachse (A) der Trägerscheibe (1) sowie die Achsen der Quetschrollen (3) und der Führungsrollen (5) parallel zueinander verlaufen und dass die Trägerscheibe (1) und/oder die drehbar auf der Trägerscheibe (1) gelagerten Quetschrollen (3) und/oder die drehbar auf der Trägerscheibe (1) gelagerten Führungsrollen (5) bei laufender Schlauchpumpe von einem Antrieb in Rotation versetzt werden.

10. Schlauchpumpe nach einem der voranstehenden Ansprüche, wobei der Betrag der relativen Winkeldifferenz (Δ - δ / Δ + δ) zwischen dem Winkelabstand Δ zwischen einer Führungsrolle und der in Förderrichtung dieser Führungsrolle vorausgehenden Quetschrolle und dem Winkelabstand δ zwischen dieser Führungsrolle und der in Förderrichtung dieser Führungsrolle nachfolgenden Quetschrolle im Bereich von 0,2 bis 0,5 liegt.

11. Schlauchpumpe nach einem der voranstehenden Ansprüche, wobei der Betrag der relativen Winkeldifferenz (Δ - δ / Δ + δ) zwischen dem Winkelabstand Δ zwischen einer Führungsrolle und der in Förderrichtung dieser Führungsrolle vorausgehenden Quetschrolle und dem Winkelabstand δ zwischen dieser Führungsrolle und der in Förderrichtung dieser Führungsrolle nachfolgenden Quetschrolle bei 0,25 liegt.

12. Schlauchpumpe nach einem der voranstehenden Ansprüche, wobei der Betrag der relativen Winkeldifferenz (Δ - δ / Δ + δ) zwischen dem Winkelabstand Δ zwischen einer Führungsrolle und der in Förderrichtung dieser Führungsrolle vorausgehenden Quetschrolle und dem Winkelabstand δ zwischen dieser Führungsrolle und der in Förderrichtung dieser Führungsrolle nachfolgenden Quetschrolle bei 0,33 liegt.

13. Schlauchpumpe nach einem der voranstehenden Ansprüche, **dadurch gekennzeichnet, dass** das im Wesentlichen kreissegmentförmige Gegenlager (4) im Bereich des Schlauchausgangs (2b) des Schlauchbetts (2) tangential nach außen ausläuft.

## Claims

1. Hose pump for conveying a fluid guided in a hose (16), with a hose bed (2) having a counter bearing (4), a carrier disc (1) rotatable relative to the counter bearing (2), a plurality of pinch rollers (3) arranged equidistantly to one another in the circumferential direction on the carrier disc (1) and a plurality of guide rollers (5) arranged equidistantly to one another in the circumferential direction on the carrier disc (1), wherein a respective guide roller (5) is arranged between two successive pinch rollers (3) in the circumferential direction of the carrier disc (1) and, when the carrier disc (1) rotates in a conveying direction (F), the pinch rollers (3) are pressing a hose inserted into the hose bed (2) against the counter bearing (4) and pinching the hose, in order to transport the fluid in the hose (16) in the conveying direction, wherein the angular distance (Δ) between a guide roller (5a) and the pinch roller (3b) preceding this guide roller (5a) in the conveying direction (F) is greater than the angular distance (δ) between this guide roller (5a) and the pinch roller (3a) following this guide roller (5a) in the conveying direction (F), **characterised in that** a cylinder (6) with a lateral surface projecting above the surface of the carrier disc (1) is arranged in the center of the carrier disc (1) coaxially to an axis of rotation (A), wherein the outer diameter (D) of the cylinder (6) extends at least approximately to the outer circumference of the pinch rollers (3) and guide rollers (5) located radially further outwards, and the radial distance between the circumferential surface of the cylinder (6) and the outer circumference of the guide rollers (5) is smaller than the diameter of the hose (16) inserted into the hose bed (2).

2. Hose pump according to claim 1, **characterised in that** the angular distance (δ) between a guide roller (5a) and the pinch roller (3a) following this guide roller (5a) in the conveying direction is less than 60° and, in particular, is at most 45°.

3. Hose pump according to claim 1, **characterised in that** the angular distance (Δ) between a guide roller (5a) and the pinch roller (3b) preceding this guide roller (5a) in the conveying direction is greater than 60° and in particular is at least 75°.

4. Hose pump according to one of the preceding claims, **characterised in that** at least three pinch rollers (3) and at least three guide rollers (5) are arranged on the carrier disc (1).

5. Hose pump according to one of the preceding claims, **characterised in that** the pinch rollers (3) are at least substantially cylindrical and have a smooth outer surface, the outer circumference of the pinch rollers (3) being formed by the smooth outer surface.

6. Hose pump according to one of the preceding claims, **characterised in that** the guide rollers (5) are at least substantially cylindrical and have a circumferential guide groove (11) on their outer circumference.

7. Hose pump according to claim 6, **characterised in that** the guide groove (11) of each guide roller (5) has a semicircular cross-section.

8. Hose pump according to one of the preceding claims, **characterised in that** a hose inserted into the hose bed (2) rests on a guide surface (2c) formed by the surface of the carrier disc (1) and is guided and held in this position by the guide rollers (5).

9. Hose pump according to one of the preceding claims, **characterised in that** the pinch rollers (3) and/or the guide rollers (5) are rotatably mounted on the carrier disc (1), wherein the axis of rotation (A) of the carrier disc (1) and the axes of the pinch rollers (3) and the guide rollers (5) run parallel to one another, and that the carrier disc (1) and/or the pinch rollers (3) mounted rotatably on the carrier disc (1) and/or the guide rollers (5) mounted rotatably on the carrier disc (1) are set in rotation by a drive when the hose pump is running.

10. Hose pump according to one of the preceding claims, wherein the amount of the relative angular difference (Δ - δ / Δ + δ) between the angular distance Δ between a guide roller and the pinch roller preceding this guide roller in the conveying direction and the angular distance δ between this guide roller and the pinch roller following this guide roller in the conveying direction is in the range from 0.2 to 0.5.

11. Hose pump according to one of the preceding claims, wherein the amount of the relative angular difference (Δ - δ / Δ + δ) between the angular distance Δ between a guide roller and the pinch roller preceding this guide roller in the conveying direction and the angular distance δ between this guide roller and the pinch roller following this guide roller in the conveying direction is 0.25

12. Hose pump according to one of the preceding claims, wherein the amount of the relative angular difference (Δ - δ / Δ + δ) between the angular distance Δ between a guide roller and the pinch roller preceding this guide roller in the conveying direction and the angular distance δ between this guide roller and the pinch roller following this guide roller in the conveying direction is 0.33

13. Hose pump according to one of the preceding claims, **characterised in that** the essentially circular segment-shaped counter bearing (4) extends tangentially outwards in the region of the hose outlet (2b) of the hose bed (2).

## Revendications

1. Pompe péristaltique pour le refoulement d'un fluide guidé dans un tube (16), avec une armature de tube (2) présentant un contre-appui (4), un disque de support (1) rotatif par rapport au contre-appui (2), une pluralité de galets d'écrasement (3) disposés sur le disque de support (1) de manière équidistante les uns des autres dans la direction circonférentielle et une pluralité de galets de guidage (5) disposés sur le disque de support (1) de manière équidistante les uns des autres dans la direction circonférentielle, dans laquelle respectivement un galet de guidage (5) est disposé entre deux galets d'écrasement (3) se succédant dans la direction circonférentielle du disque de support (1) et les galets d'écrasement (3) lorsque le disque de support (1) tourne dans une direction de refoulement (F) pressent un tube inséré dans l'armature de tube (2) avec écrasement du tube contre le contre-appui (4), afin de transporter le fluide dans le tube (16) dans la direction de refoulement, dans laquelle l'écart angulaire (Δ) entre un galet de guidage (5a) et le galet d'écrasement (3b) précédent dans la direction de refoulement (F) de ce galet de guidage (5a) est supérieur à l'écart angulaire (δ) entre ce galet de guidage (5a) et le galet d'écrasement (3a) suivant dans la direction de refoulement (F) de ce galet de guidage (5a), **caractérisée en ce qu'**un cylindre (6) faisant saillie de la surface du disque de support (1) avec une surface d'enveloppe est disposé au centre du disque de support (1) de manière coaxiale par rapport à l'axe de rotation (A), dans laquelle le diamètre extérieur (D) du cylindre (6) va au moins approximativement jusqu'à la circonférence extérieure des galets d'écrasement (3) et galets de guidage (5) situés radialement plus à l'extérieur, et l'écart radial entre la surface d'enveloppe du cylindre (6) et la circonférence extérieure des galets de guidage (5) est inférieur au diamètre du tube (16) inséré dans l'armature de tube (2).

2. Pompe péristaltique selon la revendication 1, **caractérisée en ce que** l'écart angulaire (δ) entre un galet de guidage (5a) et le galet d'écrasement (3a) suivant dans la direction de refoulement de ce galet de guidage (5a) est inférieur à 60° et en particulier égal à 45° maximum.

3. Pompe péristaltique selon la revendication 1, **caractérisée en ce que** l'écart angulaire (Δ) entre un galet de guidage (5a) et le galet d'écrasement (3b) précédent dans la direction de refoulement de ce galet de guidage (5a) est supérieur à 60° est et en particulier égal à au moins 75°.

4. Pompe péristaltique selon l'une quelconque des revendications précédentes, **caractérisée en ce qu'**au moins trois galets d'écrasement (3) et au moins trois galets de guidage (5) sont disposés sur le disque de support (1).

5. Pompe péristaltique selon l'une quelconque des revendications précédentes, **caractérisée en ce que** les galets d'écrasement (3) sont réalisés de manière au moins sensiblement cylindrique et avec une surface d'enveloppe lisse, dans laquelle la circonférence extérieure des galets d'écrasement (3) est formée par la surface d'enveloppe lisse.

6. Pompe péristaltique selon l'une quelconque des revendications précédentes, **caractérisée en ce que** les galets de guidage (5) sont au moins sensiblement cylindriques et présentent sur leur circonférence extérieure une rainure de guidage (11) périphérique dans la direction circonférentielle.

7. Pompe péristaltique selon la revendication 6, **caractérisée en ce que** la rainure de guidage (11) de chaque galet de guidage (5) présente une section transversale semi-circulaire.

8. Pompe péristaltique selon l'une quelconque des revendications précédentes, **caractérisée en ce qu'**un tube inséré dans l'armature de tube (2) repose sur une surface de guidage (2c) formée par la surface du disque de support (1) et est guidé et retenu dans cette position par les galets de guidage (5).

9. Pompe péristaltique selon l'une quelconque des revendications précédentes, **caractérisée en ce que** les galets d'écrasement (3) et/ou les galets de guidage (5) sont montés rotatifs sur le disque de support (1), dans laquelle l'axe de rotation (A) du disque de support (1) ainsi que les axes des galets d'écrasement (3) et des galets de guidage (5) s'étendent parallèlement les uns aux autres et que le disque de support (1) et/ou les galets d'écrasement (3) montés rotatifs sur le disque de support (1) et/ou les galets de guidage (5) montés rotatifs sur le disque de support (1) sont amenés en rotation par un entraînement lorsque la pompe péristaltique fonctionne.

10. Pompe péristaltique selon l'une quelconque des revendications précédentes, dans laquelle la valeur de la différence angulaire relative (Δ - δ / Δ + δ) entre l'écart angulaire Δ entre un galet de guidage et le galet d'écrasement précédent dans la direction de refoulement de ce galet de guidage et l'écart angulaire δ entre ce galet de guidage et le galet d'écrasement suivant dans la direction de refoulement de ce galet de guidage se situe dans la plage de 0,2 à 0,5.

11. Pompe péristaltique selon l'une quelconque des revendications précédentes, dans laquelle la valeur de la différence angulaire relative (Δ - δ / Δ + δ) entre l'écart angulaire Δ entre un galet de guidage et le galet d'écrasement précédent dans la direction de refoulement de ce galet de guidage et l'écart angulaire δ entre ce galet de guidage et le galet d'écrasement suivant dans la direction de refoulement de ce galet de guidage est de 0,25.

12. Pompe péristaltique selon l'une quelconque des revendications précédentes, dans laquelle la valeur de la différence angulaire relative (Δ - δ / Δ + δ) entre l'écart angulaire Δ entre un galet de guidage et le galet d'écrasement précédent dans la direction de refoulement de ce galet de guidage et l'écart angulaire δ entre ce galet de guidage et le galet d'écrasement suivant dans la direction de refoulement de ce galet de guidage est de 0,33.

13. Pompe péristaltique selon l'une quelconque des revendications précédentes, **caractérisée en ce que** le contre-appui (4) sensiblement en forme de segment de cercle se termine de manière tangentielle vers l'extérieur dans la zone de la sortie de tube (2b) de l'armature de tube (2).
